# EUROPEAN PATENT SPECIFICATION

(11) **EP 3 904 320 B1**
(45) Date of publication and mention of the grant of the patent: **04.03.2026**
(21) Application number: 19903555.1
(22) Date of filing: 20.12.2019
(51) Int. Cl.: C07C 21/20, C07C 17/389

(54) **METHOD FOR PURIFYING HEXAFLUOROBUTADIENE**
VERFAHREN ZUR REINIGUNG VON HEXAFLUORBUTADIEN
PROCÉDÉ DE PURIFICATION D'HEXAFLUOROBUTADIÈNE

(30) Priority: 28.12.2018 JP 2018247366
(43) Date of publication of application: 03.11.2021
(73) Proprietor: DAIKIN INDUSTRIES, LTD., Osaka-Shi, Osaka 530-0001 (JP)
(72) Inventor: NISHIUMI, Masami, Osaka-Shi, Osaka 5300001 (JP); NAKAI, Katsuya, Osaka-Shi, Osaka 5300001 (JP)
(74) Representative: Hoffmann Eitle
(86) International application number: PCT/JP2019/049986
(87) International publication number: WO 2020/137845

(56) References cited:
- CN-A- 108 623 432
- JP-A- 2011 136 955
- JP-B2- 3 866 663
- US-A- 3 702 886
- US-B2- 7 812 202
- SATO, MITSUO: "Outstanding Characteristics of Zeolitic Silicates and Their Uses", TETSU-TO-HAGANE, vol. 71, no. 7, 1985, pages 800 - 806, XP055721698, ISSN: 0021-1575

## Description

### Technical Field

The present disclosure relates to a method for purifying hexafluorobutadiene.

### Background Art

Hexafluorobutadiene has been widely used as an etching gas in semiconductor production processes.

Conventionally, hexafluorobutadiene is purified by removing impurities using a solid adsorbent such as a zeolite (e.g., JP-B-3866663 and JP-B-5607354). However, in the conventional technique, hexafluorobutadiene reacts with zeolite to cause isomerization of hexafluorobutadiene, which reduces the purity of hexafluorobutadiene.

JP-A-2011-136955 relates to a method for producing a high-purity fluorine-containing compound, comprising contacting the fluorine-containing compound with zeolite in which ≥ 20% of the number of ion-exchangeable ions in the zeolite have been exchanged with at least one ion selected from Ca, Mg, Ba and Li ions to remove impurities present in the fluorine-containing compound.

CN-A-108 623 432 discloses a method of purifying hexafluoro-1,3-butadiene, comprising the sequential steps of (A) subjecting raw hexafluoro-1,3-butadiene (purity 80-98%) to primary adsorption in the gas phase using activated carbon loaded with an alkali and/or alkaline earth metal compound as adsorption agent, (B) performing secondary adsorption using a molecular sieve loaded with a compound of an alkali metal, a rare earth metal or a transition metal as adsorption agent, and (C) rectifying in rectifying column.

US 7,821,202 describes a process for producing hexafluoro-1,3-butadiene comprising ((1) reacting a compound having four carbon atoms each which bonds to an atom selected from Cl, Br and I with fluorine gas in the presence of a diluting gas in the gas phase, to prepare a mixture containing a product (A), and (2) eliminating halogens excluding F with a metal from the product (A) in the presence of a solvent, thereby preparing a mixture containing hexafluoro-1,3-butadiene.

### Summary of Invention

### Technical Problem

An object of the present disclosure is to purify hexafluorobutadiene to a high purity while inhibiting the isomerization of hexafluorobutadiene.

### Solution to Problem

The present invention provides a method for purifying hexafluorobutadiene, comprising contacting a composition containing hexafluorobutadiene with a zeolite having (i) an SiO₂/Al₂O₃ molar ratio of 4.0-8.0 and (ii) an average pore size of 5-9 Å.

Also, the present invention provides a method for producing a composition containing hexafluorobutadiene, comprising contacting a composition containing hexafluorobutadiene with a zeolite having (i) an SiO₂/Al₂O₃ molar ratio of 4.0-8.0 and (ii) an average pore size of 5-9 Å, wherein the purity of the hexafluorobutadiene in the composition after the contact with the zeolite is ≥ 99.99 vol.%.

Preferred embodiments of the invention are as defined in the appended dependent claims and/or in the following detailed description.

### Advantageous Effects of Invention

According to the present invention, the isomerization of hexafluorobutadiene can be inhibited, and the hexafluorobutadiene can be purified to a high purity.

### Description of Embodiments

As a result of extensive research, the present inventors found that the isomerization of hexafluorobutadiene can be inhibited by contacting a composition containing hexafluorobutadiene with a zeolite having an SiO₂/Al₂O₃ molar ratio of 4.0-8.0 and an average pore size of 5-9 Å, thereby purifying the hexafluorobutadiene to a high purity.

The present invention was accomplished as a result of further research based on such findings.

The present invention includes the following embodiments.

Herein, hexafluorobutadiene refers to hexafluoro-1,3-butadiene.

### (1) Method for purifying hexafluorobutadiene

The present method for purifying hexafluorobutadiene comprises the step of contacting a composition containing hexafluorobutadiene with a zeolite having an SiO₂/Al₂O₃ molar ratio of 4.0-8.0 and an average pore size of 5-9 Å.

In the above step, an impurity other than the hexafluorobutadiene is adsorbed onto the zeolite to remove the impurity from the composition before the contact with the zeolite.

According to the present invention, in the step, the purity of the hexafluorobutadiene in the composition after the contact with the zeolite is ≥ 99.99 vol.%.

The present method for purifying hexafluorobutadiene has an advantage such that the isomerization of hexafluorobutadiene is inhibited and the hexafluorobutadiene is purified to a high purity by contacting the composition containing hexafluorobutadiene with the zeolite having an SiO₂/Al₂O₃ molar ratio of 4.0-8.0 and an average pore size of 5-9 Å.

### (1-1) Composition containing hexafluorobutadiene

Hexafluorobutadiene is used as an etching gas for silicon oxide and related materials in the production of semiconductors. The hexafluorobutadiene used in the semiconductor industry is required to have extremely high purity.

The composition containing hexafluorobutadiene contains an impurity that is introduced during the production of hexafluorobutadiene. Examples of the impurity include intermediates, isomers, and by-products (e.g., HF, hydrofluorocarbons, hydrochlorofluorocarbons, chlorofluorocarbons, and fluoroalkenes).

The composition containing hexafluorobutadiene contains an impurity that is introduced during the use of hexafluorobutadiene. Examples of the impurity include HF, SiF₄, and O₂ when the hexafluorobutadiene is used as an etching gas for the production of semiconductors. Examples of the impurity include moisture when the hexafluorobutadiene is used as a refrigerant for refrigeration equipment.

When e.g. water or HF exist as an impurity, especially when a fluorine-containing compound is chemically unstable due to having a reactive moiety such as a double bond, alteration (decomposition, polymerization, or isomerization) of the fluorine-containing compound may occur, leading to a vicious cycle of further decrease in purity.

Commercially available hexafluorobutadiene typically has a purity of 99.0-99.9 vol.%; and includes a partially fluorinated and chlorinated isomer of butadiene, a butadiene dimer, and a mixture of a starting material and a solvent.

Hexafluorobutadiene having a higher purity is needed in the semiconductor industry.

### (1-2) Zeolite having an SiO₂/Al₂O₃ molar ratio of 4.0-8.0 and an average pore size of 5-9 Å

Zeolites are one of the clay minerals. They are hydrous aluminosilicates that contain alkali or alkaline earth metals. Zeolites are composed of a rigid anionic framework with regular channels (tubular pores) and cavities (cavities).

Zeolites are typically represented by the composition of (M^{I},M^{II}_{1/2})ₘ(AlₘSiₙO₂₍ₘ₊ₙ₎)ₓH₂O (n≥m) (M^{I}: Li⁺, Na⁺, K⁺, etc., M^{II}: Ca²⁺, Mg²⁺, Ba²⁺, etc.). The cations compensate for the negative charge of the aluminosilicate framework.

There is no limitation on the cation in the zeolite; for example, H⁺, Li⁺, Na⁺, K, Ca²⁺, Mg²⁺ and Ba²⁺ are usually used.

The basic unit of the structure is a tetrahedral structure of SiO₄ or AlO₄ (TO₄ tetrahedron in combination). These structures are connected forever in the three-dimensional direction to form a crystal. The crystal of zeolite is porous, and typically has a pore diameter of 0.2-1.0 nm. The zeolite has a molecular sieving effect (molecular sieve), which means that a molecule having a size larger than the pore size of the zeolite cannot enter. The zeolite has properties such as solid acidity, ion exchange capacity, catalytic capacity, and adsorption capacity, in addition to the molecular sieving effect due to the pore derived from its framework structure.

In the present invention, a zeolite having an SiO₂/Al₂O₃ molar ratio of 4.0-8.0 is used because the isomerization of hexafluorobutadiene can be inhibited, and the hexafluorobutadiene can be purified to a high purity. In the present invention, it is preferable to use a zeolite having an SiO₂/Al₂O₃ molar ratio of 5.0-7.0.

If the SiO₂/Al₂O₃ molar ratio is < 4.0, the polarity of the zeolite increases, and the zeolite itself is easily degraded by the reaction with HF in the composition containing hexafluorobutadiene. If the SiO₂/Al₂O₃ molar ratio is > 8.0, the polarity of the zeolite is reduced, and the adsorption amount of water in the composition containing hexafluorobutadiene decreases.

In the present invention, because the isomerization of hexafluorobutadiene can be inhibited and the hexafluorobutadiene can be purified to a high purity, a zeolite having an average pore size of 5-9Å is used. Such a zeolite is preferably porous. Such a zeolite is commercially available.

Impurities to be adsorbed and removed by the zeolite include, *inter alia,* those containing water, those containing HF, those containing hydrocarbons; those containing carbon halide compounds; those containing halogenated hydrocarbon compounds; and those containing isomers of fluorine-containing compounds to be contacted with the zeolite, such as positional isomers of carbon-carbon double bonds and cyclic isomers of carbon-carbon double bonds. Examples of the aforementioned impurities include heptafluorobutene, chloroheptafluorobutene, methyl iodide and chlorotrifluoroethylene.

### (1-3) Step of contacting the composition containing hexafluorobutadiene with zeolite

The present invention includes the step of contacting the composition containing hexafluorobutadiene with a zeolite having an SiO₂/Al₂O₃ molar ratio of 4.0-8.0 and an average pore size of 5-9 Å. Herein, contacting the composition with the zeolite means allowing the composition to pass through e.g. a column filled with the zeolite, or introducing the composition into a container filled with the zeolite.

The zeolite is used as a material for adsorbing and removing an impurity, and the composition containing hexafluorobutadiene is brought into contact with (allowed to pass through) the zeolite to remove an impurity contained in the composition.

For effective adsorption and removal of impurities, it is preferable to bring the composition containing hexafluorobutadiene into contact with the zeolite in a mass ratio of (100:1)-(1:10), more preferably (50:1)-(1:5), and even more preferably (10:1)-(1:3). The amount of the zeolite to be used may be such that the amount of the composition containing hexafluorobutadiene is 10-100 g when e.g. a stainless cylinder is filled with 10 g of zeolite (zeolite filling).

The zeolite may be activated and treated before the use thereof. As the condition for the activation treatment, a drying treatment in which heating is conducted in a vacuum (13,33-0,1333 Pa (10⁻¹ mmHg to 10⁻³ mmHg)) at a temperature in the range of 300-350°C overnight can be exemplified. In the present disclosure, zeolites that do not undergo the aforementioned activation treatment can also be suitably used.

There is no limitation on the form of the zeolite to be used. The composition containing hexafluorobutadiene may pass through a device filled with the zeolite; or the composition may be introduced into a device filled with the zeolite, and then removed after a predetermined time has passed.

There is no limitation on the temperature at which the composition containing hexafluorobutadiene is brought into contact with the zeolite. The temperature may be determined considering the boiling point of hexafluorobutadiene contained in the composition including hexafluorobutadiene. In general, contact at a low temperature is preferable because a side reaction such as isomerization during the contact is inhibited. The temperature is preferably in the range of -50°C to 100°C.

There is no limitation on the time for bringing the composition containing hexafluorobutadiene into contact with the zeolite.

The zeolite to be used may be in the form of powders, granules, or pellets; or may be used as a molded body. From the industrial perspective, the zeolite to be used is preferably a molded body. Although there is no limitation on the shape of the molded body, a cylindrical shape having a diameter of 0.5-5 mm and a length of 1-15 mm, or a spherical shape having a diameter of 0.5-10 mm is preferably used.

The method for producing the molded body of the zeolite is not limited. For example, a conventional known method using kaolin clay as a binder can be used.

According to the present method for purifying hexafluorobutadiene, by contacting the composition containing hexafluorobutadiene with a zeolite having an SiO₂/Al₂O₃ molar ratio of 4.0-8.0 and an average pore size of 5-9 Å, an impurity other than the hexafluorobutadiene can be adsorbed onto the zeolite, thereby removing the impurity from the composition.

According to the present method for purifying hexafluorobutadiene, in the composition after purification, i.e., in the composition after the contact with the zeolite, the purity of the hexafluorobutadiene can be adjusted to ≥ 99.99 vol.%, and can be particularly adjusted to ≥ 99.995 vol.% after purification.

According to the present invention, by effectively removing various impurities in the composition containing hexafluorobutadiene, the composition from which the impurities have been removed can be suitably used as an etching gas for the production of semiconductors, thus allowing to inhibit corrosion and premature degradation of equipment.

### (2) Method for producing the composition containing hexafluorobutadiene

The present method for producing a composition containing hexafluorobutadiene comprises the step of contacting the composition containing hexafluorobutadiene with a zeolite having an SiO₂/Al₂O₃ molar ratio of 4.0-8.0 and an average pore size of 5-9 Å, wherein the purity of the hexafluorobutadiene in the composition after the contact with the zeolite is ≥ 99.99 vol.%.

In the above step, it is preferable to adsorb an impurity other than the hexafluorobutadiene onto the zeolite, and remove the impurity from the composition before the contact with the zeolite.

The purity of hexafluorobutadiene in the composition after the contact with the zeolite can be particularly adjusted to ≥ 99.995 vol.%.

In the present method for producing a composition containing hexafluorobutadiene, the zeolite to be used, the composition containing hexafluorobutadiene that is brought into contact with the zeolite, and the like are as explained in the present purification method of hexafluorobutadiene.

### (3) Composition containing hexafluorobutadiene

In the present composition, the purity of hexafluorobutadiene is ≥ 99.995 vol.%.

The composition is used as an etching gas or a building block for organic synthesis. The composition can be effectively used for various applications such as building blocks for organic synthesis; as well as etching gases for forming state-of-the-art microstructures, such as semiconductors and liquid crystals.

The building block for organic synthesis refers to a substance that can be a precursor for a compound having a highly reactive framework. For example, by the reaction of the present composition with a fluorine-containing organosilicon compound such as CF₃Si(CH₃)₃, a fluoroalkyl group such as a CF₃ group is introduced into the composition, thereby converting the composition into a substance that can become a detergent or a fluorine-containing pharmaceutical intermediate.

### Examples

The present invention is explained in detail below with reference to the following Examples.

### Measurement of purity of hexafluorobutadiene and impurity concentration

The purity was measured using the following measuring device and conditions.

Measuring device: Gas chromatography (an FID detector is used)
Measuring conditions: Column/GS-GasPro, Column temperature/40°C Calculation of purity: Quantified by a calibration curve using a hexafluorobutadiene standard gas.

### (1) Method for purifying hexafluorobutadiene in the Examples

A composition containing hexafluorobutadiene (hexafluoro-1,3-butadiene) (purity of hexafluorobutadiene: 99.875 vol.%) was brought into contact with a zeolite having an SiO₂/Al₂O₃ molar ratio of 4.0-8.0 and an average pore size of 5-9 Å.

### Example 1

50 g of a composition containing hexafluorobutadiene was introduced into a stainless steel cylinder (100 mL) filled with 10 g of zeolite having an SiO₂/Al₂O₃ molar ratio of 6.1 and an average pore size of 5Å, and the cylinder was kept at -18°C for 20 hours. Gas chromatography analysis indicated that the purity of hexafluorobutadiene in the composition after contact with the zeolite was 99.997 vol.%. Since the concentration of the isomer of hexafluorobutadiene was below the detection limit, it was determined as 0 vol.%.

### Example 2

50 g of a composition containing hexafluorobutadiene was introduced into a stainless steel cylinder (100 mL) filled with 10 g of zeolite having an SiO₂/Al₂O₃ molar ratio of 5.5 and an average pore size of 9Å, and the cylinder was kept at -18°C for 20 hours. Gas chromatography analysis indicated that the purity of hexafluorobutadiene in the composition after contact with the zeolite was 99.995 vol.%. Since the concentration of the isomer of hexafluorobutadiene was below the detection limit, it was determined as 0 vol.%.

### (2) Method for purifying hexafluorobutadiene in the Comparative Examples

The composition containing hexafluorobutadiene (purity of hexafluorobutadiene: 99.875 vol.%) was brought into contact with a zeolite having an SiO₂/Al₂O₃ molar ratio of 4.0.8.0.

### Comparative Example 1

50 g of a composition containing hexafluorobutadiene was introduced into a stainless steel cylinder (100 mL) filled with 10 g of zeolite having an SiO₂/Al₂O₃ molar ratio of 9.2 and an average pore size of 4Å, and the cylinder was kept at -18°C for 20 hours. Gas chromatography analysis indicated that the purity of hexafluorobutadiene in the composition after contact with the zeolite was 99.151 vol.%. The concentration of the isomer of hexafluorobutadiene was detected as 0.724 vol.%.

### Comparative Example 2

50 g of a composition containing hexafluorobutadiene was introduced into a stainless steel cylinder (100 mL) filled with 10 g of zeolite having an SiO₂/Al₂O₃ molar ratio of 1.9 and an average pore size of 3Å, and the cylinder was kept at -18°C for 20 hours. Gas chromatography analysis indicated that the purity of hexafluorobutadiene in the composition after contact with the zeolite was 99.864 vol.%. The concentration of the isomer of hexafluorobutadiene was detected as 0.011 vol.%.

### Comparative Example 3

50 g of a composition containing hexafluorobutadiene was introduced into a stainless steel cylinder (100 mL) filled with 10 g of zeolite having an SiO₂/Al₂O₃ molar ratio of 2.6 and an average pore size of 3Å, and the cylinder was kept at -18°C for 20 hours. Gas chromatography analysis indicated that the purity of hexafluorobutadiene in the composition after contact with the zeolite was 99.782 vol.%. The concentration of the isomer of hexafluorobutadiene was detected as 0.093 vol.%.

### Comparative Example 4

50 g of a composition containing hexafluorobutadiene was introduced into a stainless steel cylinder (100 mL) filled with 10 g of zeolite having an SiO₂/Al₂O₃ molar ratio of 2.1 and an average pore size of 5Å, and the cylinder was kept at -18°C for 20 hours. Gas chromatography analysis indicated that the purity of hexafluorobutadiene in the composition after contact with the zeolite was 99.868 vol.%. The concentration of the isomer of hexafluorobutadiene was detected as 0.007 vol.%.

### (3) Summary of method for purifying hexafluorobutadiene

Semiconductor integrated circuit devices undergo miniaturization of circuit patterns along with their acceleration and high integration. Hexafluorobutadiene is an essential gas for etching the finest contact holes.

According to the present method for purifying hexafluorobutadiene, by contacting the composition containing hexafluorobutadiene with a zeolite having an SiO₂/Al₂O₃ molar ratio of 4.0-8.0 and an average pore size of 5-9 Å, an impurity other than the hexafluorobutadiene is adsorbed onto the zeolite to remove the impurity from the composition before the contact with the zeolite, thus adjusting the purity of hexafluorobutadiene in the composition after the contact with the zeolite to ≥ 99.99 vol.%.

According to the present method for purifying hexafluorobutadiene, hexafluorobutadiene can be purified to a high purity while inhibiting the isomerization of hexafluorobutadiene. Highly purified hexafluorobutadiene is advantageous for etching fine contact holes and forming fine circuit patterns in semiconductor integrated circuit devices.

## Claims

1. A method for purifying hexafluorobutadiene, comprising contacting a composition containing hexafluorobutadiene with a zeolite having (i) an SiO₂/Al₂O₃ molar ratio of 4.0-8.0 and (ii) an average pore size of 5-9 Å.

2. The method of claim 1, wherein an impurity other than the hexafluorobutadiene is adsorbed onto the zeolite to remove the impurity from the composition.

3. The method of claim 1 or 2, wherein the purity of the hexafluorobutadiene in the composition after the purification is ≥ 99.99 vol.%, and preferably ≥ 99.995 vol.%.

4. A method for producing a composition containing hexafluorobutadiene, comprising contacting a composition containing hexafluorobutadiene with a zeolite having (i) an SiO₂/Al₂O₃ molar ratio of 4.0-8.0 and (ii) an average pore size of 5-9 Å, wherein the purity of the hexafluorobutadiene in the composition after the contact with the zeolite is ≥ 99.99 vol.%.

5. The method of claim 4, wherein an impurity other than the hexafluorobutadiene is adsorbed onto the zeolite to remove the impurity from the composition before the contact with the zeolite.

6. The method of claim 4 or 5, wherein the purity of the hexafluorobutadiene in the composition after the contact with the zeolite is ≥ 99.995 vol.%.

## Patentansprüche

1. Verfahren zur Reinigung von Hexafluorbutadien, umfassend das Inkontaktbringen einer Hexafluorbutadien enthaltenden Zusammensetzung mit einem Zeolith mit (i) einem SiO₂/Al₂O₃-Molverhältnis von 4,0 bis 8,0 und (ii) einer durchschnittlichen Porengröße von 5 bis 9 Å.

2. Verfahren gemäß Anspruch 1, wobei eine andere Verunreinigung als das Hexafluorbutadien an dem Zeolith adsorbiert wird, um die Verunreinigung aus der Zusammensetzung zu entfernen.

3. Verfahren gemäß Anspruch 1 oder 2, wobei die Reinheit des Hexafluorbutadiens in der Zusammensetzung nach der Reinigung ≥ 99,99 Vol.-% und vorzugsweise ≥ 99,995 Vol.-% beträgt.

4. Verfahren zur Herstellung einer Hexafluorbutadien enthaltenden Zusammensetzung, umfassend das Inkontaktbringen einer Hexafluorbutadien enthaltenden Zusammensetzung mit einem Zeolith mit (i) einem SiO_{2/}Al₂O₃-Molverhältnis von 4,0 bis 8,0 und (ii) einer durchschnittlichen Porengröße von 5 bis 9 Å, wobei die Reinheit des Hexafluorbutadiens in der Zusammensetzung nach dem Kontakt mit dem Zeolith ≥ 99,99 Vol.-% beträgt.

5. Verfahren gemäß Anspruch 4, wobei eine andere Verunreinigung als das Hexafluorbutadien an dem Zeolith adsorbiert wird, um die Verunreinigung aus der Zusammensetzung zu entfernen vor dem Kontakt mit dem Zeolith.

6. Verfahren gemäß Anspruch 4 oder 5, wobei die Reinheit des Hexafluorbutadiens in der Zusammensetzung nach dem Kontakt mit dem Zeolith ≥ 99,995 Vol.-% beträgt.

## Revendications

1. Procédé de purification de l'hexafluorobutadiène, comprenant la mise en contact d'une composition contenant de l'hexafluorobutadiène avec une zéolite ayant (i) un rapport molaire SiO₂/Al₂O₃ de 4,0 à 8,0 et (ii) une taille moyenne de pores de 5 à 9 Å.

2. Le procédé selon la revendication 1, dans lequel une impureté autre que l'hexafluorobutadiène est adsorbée sur la zéolite afin d'éliminer l'impureté de la composition.

3. Le procédé selon la revendication 1 ou 2, dans lequel la pureté de l'hexafluorobutadiène dans la composition après purification est ≥ 99,99 % en volume, et de préférence ≥ 99,995 % en volume.

4. Le procédé de production d'une composition contenant de l'hexafluorobutadiène, comprenant la mise en contact d'une composition contenant de l'hexafluorobutadiène avec une zéolite ayant (i) un rapport molaire SiO₂/Al₂O₃ de 4,0 à 8,0 et (ii) une taille moyenne de pores de 5 à 9 Å, dans lequel la pureté de l'hexafluorobutadiène dans la composition après le contact avec la zéolite est ≥ 99,99 % en volume.

5. Le procédé selon la revendication 4, dans lequel une impureté autre que l'hexafluorobutadiène est adsorbée sur la zéolite afin d'éliminer l'impureté de la composition avant le contact avec la zéolite.

6. Le procédé selon la revendication 4 ou 5, dans lequel la pureté de l'hexafluorobutadiène dans la composition après le contact avec la zéolite est ≥ 99,995 % en volume.
